# EUROPEAN PATENT SPECIFICATION

(11) **EP 1 520 049 B1**
(45) Date of publication and mention of the grant of the patent: **26.12.2007**
(21) Application number: 03763984.6
(22) Date of filing: 10.07.2003
(51) Int. Cl.: C12Q 1/68, C12N 15/10

(54) **NUCLEIC ACID AMPLIFICATION METHOD**
NUKLEINSAURE VERVIELFAELTIGUNGSVERFAHREN
METHODE D'AMPLIFICATION D'ACIDE NUCLEIQUE

(30) Priority: 10.07.2002 GB 0216026
(43) Date of publication of application: 06.04.2005
(73) Proprietor: Cambridge Biotechnology Ltd., Cambridge CB1 1HW (GB)
(72) Inventor: DIXON, Alastair NeuroSolutionse Limited, Coventry CV4 7ZS (GB); SKYNNER, Michael University of Cambridge, Cambridge CB12 1QJ (GB)
(74) Representative: Perry, Robert Edward
(86) International application number: PCT/GB2003/002989
(87) International publication number: WO 2004/007766

(56) References cited:
- EP-A- 1 275 738
- WO-A-00/08208
- WO-A-01/06004

## Description

### Field of the Invention

This invention relates to a nucleic acid amplification method.

### Background of the Invention

WO01/06004 discloses a method for increasing the number of polynucleotides containing sequences corresponding to a mRNA species present in a sample. This method comprises 3 steps, i.e. reverse transcription of the mRNA species using a first heeled primer population, to provide first cDNA strands; synthesis of second cDNA strands from the first, using a second heeled primer population; and amplification of the first and second cDNA strands. The heel sequence of the primers used in the first and/or second steps contains a RNA polymerase promoter site. The primers used in the amplification comprise at least a portion of the first heel sequence and a portion of the second heel sequence. Either or each ofthe heel sequences preferably includes the nucleotide sequence of a rare restriction enzyme cleavage site; the polynucleotides that are produced may include concatomers, but can be treated with an agent that cleaves at this cleavage site, without affecting the desired amplicons.

### Summary of the Invention

The present invention is based on the discovery that the procedure disclosed in WO01/06004 can be simplified. According to the present invention, a method for increasing the number of polynucleotides containing sequences corresponding to a mRNA species present in a sample, comprises the steps of:
(i) in a single step, reverse transcription of the mRNA species using a heeled 5'-amplification primer and a heeled 3'-amplification primer, each heeled primer comprising a heel sequence including a unique sequence, and either or each heel sequence includes a RNA polymerase promoter site, and the heeled 5'-amplification primer includes a variable sequence, and the heeled 3' amplification primer primes at the 3' polyA site in the transcripts
   whereby the RNA is reverse-transcribed to produce double-stranded cDNA and then multiple cDNAs according to the variable sequence; and
(ii) amplification of the cDNA using primers sufficiency identical to the unique sequences within the heel sequences of the heeled 5'-amplification primer and the heeled 3'-amplification primer.

It will be evident that, in the present invention, reverse transcription and production of multiple cDNAs are conducted in one step. While the present invention shares with the known procedure the use of a randomer sequence, so that the use of appropriate populations of varied sequences gives multiple cDNAs, the novel procedure is simpler. The reverse transcription proceeds more efficiently, and fewer amplification cycles are required.

Further, by comparison with the known procedure, the need for rare restriction sites is avoided. Another advantage is that the production of complex products is minimised, due in part to the use of unique sequences in FAP and TAP which are absent from the genome being investigated. Moreover, while the known procedure uses a single primer to amplify the products after RT and second strand synthesis, the present invention provides the significant advantage that two separate primers ofunique sequence are used.

The present invention not only provides greater amplification but also greater flexibility in use. For example, it readily allows the inclusion of specific restriction sites, for manufacturing subtracted normalised and enriched cDNA libraries. It also allows the inclusion of specific restriction sites for lambda cloning, for the manufacture of single cell libraries. Again, specific restriction sites can be included, for fragmentation, for use as probes on the microarrays and filters. The procedure can readily be linked to a protocol for laser capture microdissection, e.g. as described by Fend et al, Am. J. Pathol. (1999) 154(1):61-6.

An embodiment of the invention comprises a third step, i.e. (iii) *in vitro* transcription, to produce RNA run-offs from either end of the amplicons, e.g. using T3 RNA polymerase or T7 RNA polymerase. This is particularly suitable for use when the quantity of starting RNA is low or the number of available cells is small.

### Description of Preferred Embodiments

The present invention (described herein as "MEX") is a technology for molecular analysis of gene expression in small numbers of cells, even down to the single cell level. MEX products are designed to be directly integrated and compatible with a raft of downstream molecular technologies including single-cell PCR, the construction and screening of subtracted expression libraries, microarray analysis and target validation.

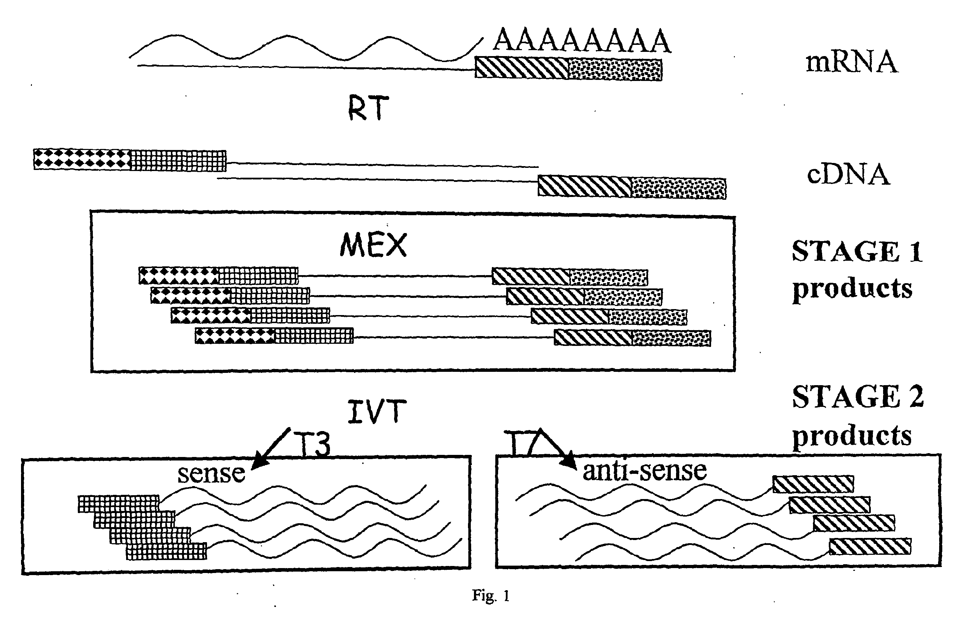

Without wishing to be bound by theory, MEX amplification occurs in two stages: a PCR (Polymerase Chain Reaction) step followed, if required, by an IVT (*in vitro* Transcription) step. A summary of the mechanics underlying MEX is shown in Figure 1. Briefly, this drawing shows a first stage in which reverse transcription (RT) specific sequences are incorporated at both 5' and 3' ends ofthe cellular cDNA population. These sequences are used as priming sites for MEX-PCR primers (respectively shown as diamond and stippled boxes, in Fig.1) in stage one amplification. Subsequently RNA polymerase promoters (respectively shown as striped and cross-hatched boxes, in Fig. 1), contained within the MEX RT primers, are used for the production of stage 2 MEX products. These are strand-specific RNA species that can be used in multiple downstream molecular applications.

In MEX stage one, the cDNA complement of a cell population is tagged at both 5' and 3' ends with, say, proprietary primer sequences. These tagged products are subsequently amplified using limited numbers of cycles of PCR. This provides sufficient material for downstream analysis from as few as a 1000 cells (10-100ng total RND); these are referred herein to as MEX stage one products.

MEX stage one products are dependant upon the incorporation of MEX-RT priming sites within the cDNA pool and are of a similar size to transcripts in the original RNA population prior to MEX. This illustrates that in MEX full-length cDNAs are produced and amplified. In addition, bioinfomatic analysis of cloned MEX products has shown that 100% of products contain MEX-RT primers at appropriate sites in the transcript (n=43), i.e. the 3' MEX primer primes precisely at the 3' poly A site in the transcript and the 5' primer is contained at the 5' end of the transcript.

In a particular embodiment, the heeled 3'-amplification primer initiates reverse transcription and first round cDNA synthesis through binding of a (T)₂₀ sequence, contained in the 3'-terminus of the primer, to polyA tails in the mRNA population. This primer contains a heel sequence for subsequent PCR amplification and a nested RNA polymerase promoter site (for T7 RNA polymerase) for subsequent *in vitro* transcription reactions. A second primer, the heeded 5'-amplification primer, is also included in the RT reaction; this initiates second round cDNA synthesis through semi-randomly priming from the first strand cDNA via a (N)₁₅ sequence incorporated at the 3'-terminus of the primer. This primer also contains a heel sequence for subsequent PCR amplification (NB. this heel sequence is distinct from that in the 3'-primer) and a nested RNA polymerase promoter site (for T3 RNA polymerase) for subsequent *in vitro* transcription reactions. The heel sequences are unique sequences as defined by their absence from the current genome databases.

In this way, a population of mRNAs is converted to double-stranded cDNAs that are anchored at their 3'-termini to the TAP and at their 5'-termini to the FAP. The cDNA population therefore contains the following species of MEX fragments (where Gene X represents any gene that has been reverse transcribed):
FAP SEQUENCE. T3 RNA POLYMERASE SEQUENCE (N)₁₅-GENE X-(T)₂₀-T7 RNA POLYMERASE-TAP SEQUENCE

When additional amplification is required, e.g. due to the limiting nature of the available RNA in the starting sample, an IVT reaction can be performed on stage one MEX products to extend the sensitivity of this technology. This is achieved through the incorporation of a RNA polymerase site within the proprietary MEX-RT cDNA synthesis primers and extends the range of detection to the single cell level (~10-100pg total RNA). This technique also allows the production of strand-specific probes (both sense and anti-sense) from the MEX amplicon pool through differential tagging of the 5' primer with a T3 site and the 3' primer with a T7 site.

The power of MEX is derived from its ability to amplify reliably, simply, reproducibly and in a representative manner small quantities of mRNA. Nevertheless, the true potential of this technology may best be realized through linking it to a series of upstream and downstream applications. These include upstream cell harvesting techniques such as Laser Capture Microdissection (LCM) and Patch Clamp Harvesting (PCH), and downstream molecular applications that use the MEX products as starting points.

Thus, it is known that the scattered location of diseased cells in many tissues and the difficulty of purifying these cells to homogeneity by conventional techniques has led to the development of LCM. Using this approach it is now possible to visually identify and harvest cells of interest in moderate numbers with a high degree of accuracy. These cells can be processed to isolate the RNA and this amplified using MEX.

Further, MEX products may be designed to be compatible with a range of high throughput molecular techniques. Design features in the MEX primers include the incorporation of vector-compatible restriction sites for the construction of PCR-subtracted libraries and the ability to produce strand-specific probes for screening microarrays and Affymetrix chips.

As shown in more detail below, MEX has been used successfully to produce subtracted PCR libraries. These have been compared to conventionally produced libraries and shown to be extremely similar in composition. In this way, it is possible to amplify the RNA contents from two small populations of cells, to subtract one population from the other and produce paradigm-specific libraries with a high degree of enrichment for differentially expressed genes.

An efficient method for screening the populations of products contained within MEX-subtracted libraries or within the raw MEX amplicon pools is through cloning these products and immobilising them on a substrate, e.g. by coupling them to glass microarrays as probes. In addition and in parallel, raw amplicons and libraries can be used as labeled targets to screen these and other microarrays.

An important facet of any amplification technology is the requirement that post-amplification the representation of RNA species should reflect that in the unamplified material. This allows comparative analyses between samples to be performed. MEX maintains the representation of the original starting population of RNA following amplification. This is true whether the starting samples are similar or divergent and compares well with the representation in the original pre-amplification samples. Equally, representation is maintained even at low concentrations of starting material.

While bearing in mind the key distinctions between the present invention and the method disclosed in WO01/06004, the disclosure of the latter document is relevant in many respects. For example, that document discloses certain relevant materials and process conditions, and which can readily be adapted for the purposes of this invention by one of ordinary skill in the art.

The following Examples illustrate the invention. As above, "FAP" and "TAP" are used as abbreviations for 5'- and 3'-amplification primers, respectively.

### Example 1 MEX RT reaction

In this step, specific priming sites for PCR amplification were engineered into appropriate ends of the cDNA population.

10 µl of total RNA (prepared either by standard molecular biology protocols from cells/tissues/Laser Capture Microscopy cell harvests or from direct patch clamp harvesting) dissolved in water was added to 1 µl of each of heeled 5'-amplification primer, and heeled 3-amplification primer, each at 10 µM. The heeled 5'-amplification primer is ACT CCG GGA ACC ATA GTG GCA CTC CTA ATT AAC CCT CAC TAA AGG GAG ATC GTAC (N)₁₅, of which bases 1-25 constitute the primer (FAP), bases 26-46 the T3 RNA polymerase promoter, bases 48-51 (GATC) a Sau3A restriction site and bases 52-55 (GTAC) a Rsal restriction site. The heeled 3-amplification primer sequence is CAA GCA TTC AGT ATC TCG ACT GTA ATA CGA CTC ACT ATA GGG AGA GAT CGT AC (T)₂₀, of which bases 1-22 constitute the primer (TAP), bases 22-45 the T7 RNA polymerase promoter, and bases 46-53 the same restriction sites. This was heated to 70°C for 5 minutes before snap-cooling on ice for 2 minutes.

4 µl of 5x Reverse Transcription buffer (Superscript II kit, Invitrogen), 2 µl of 0.1 M dithiothreitol (DTT, Superscript II kit, Invitrogen), 1 µl of dNTPs (10 mM mixture of each of dATP, dTTP, dGTP and dCTP), and 1 µl of Superscript II reverse transcriptase enzyme (Superscript II at 200U/µl, Invitrogen) were added, and the reaction incubated at 42°C for 1 hour.

### MEX amplification reaction

In this step, the reverse-transcribed MEX-RT products were amplified for downstream molecutar applications by PCR.

5 µl of the RT reaction was amplified in a 100 µl PCR by the addition of the following reagents: 10 µl Advantage II PCR buffer (Clontech) 1 µl of dNTPs (10mM mixture of each of dATP, dTTP, DGTP and dCTP), 1 µl FAP, i.e. bases 1-25 of heeled 5'-amplification primer defined above, at 10 µM, and 1 µl TAP, i.e. bases 1-22 of heeled 3'-amplification primer defined above, at 10 µM and 77 µl of sterile water.

Cycling parameters were as follows. 95 ° C for 1 minute, for one cycle, then up to. 24 cycles of 95°C for 15 seconds, 65°C for 30 seconds, 68°C for 6 minutes.

### Example 2 MEX from control BNAs

Approx. 100 ng of total brain RNA was reverse-transcribed according to the MEX RT/amplification protocol of Example 1. This was serially diluted and subjected to MEX amplification for either 9 cycles or 15 cycles. In addition, a control, where no amplification was performed, was also included. PCR was performed to determine the degree of amplification of actin mRNA transcripts in the cDNA and MEX amplified pools.

Actin transcripts could only be detected in unamplified cDNAs at a concentration of 50 ng of total RNA equivalent. Transcripts could not be detected at cDNA concentrations below 5 pg. In contrast, actin could be detected in the same cDNA samples at concentrations as low as 0.5 pg after 9 cycles of MEX amplification and 50.fg after 15 cycles of MEX amplification.

This experiment demonstrates that MEX amplification of cDNAs extends the range of detection beyond that where detection is possible by conventional RT PCR alone.

### Example 3 MEX from Laser Capture Cells

G-1 cultured cells were grown on glass microscope slides and processed for Laser Capture Microdissection (LCM). Defined numbers of cells were harvested, and RNA extracted using the Stratagene mico-RNA extraction kit. This RNA was reverse-transcribed using MEX RT primers and then subjected to MEX amplification for either 15 or 21 cycles. A proportion of the cDNA was also retained for analysis without MEX amplification. PCR was performed, using primers specific for actin, as the final assay of the efficiency of the MEX amplification.

Actin sequence was detected in as few as 50 cells after 15 cycles of MEX amplification and in as few as 10 cells after 21 cycles of MEX amplification. In contrast, actin sequence was undetectable in samples that were not subject to MEX pre-amplification. This demonstrates that MEX can be used to link molecular techniques to LCM.

### Example 4 MEX linked to in vitro transcription

Approx. 100 ng of total RNA was reverse-transcribed using MEX primers, serially diluted and subsequently MEX-amplified for three, six or nine cycles. These products were *in vitro* transcribed (IVT) using theMegascribe kit (Ambion) and reverse-transcribed for a second time using a conventional (dT)₁₈ primer and Superscripts II (Invitrogen). To gauge the relative efficiency of the amplifications, 30 cycles of gene-specific PCR for the actin sequence were performed on these products. The combination of three cycles of MEX, when used in conjunction with IVT, was sufficient to enable the identification of actin transcripts in 5 pg of total RNA. The application of an additional three cycles of MEX prior to IVT enabled this detection window to be extended to 50 fg of total RNA. For comparison, 15 cycles of MEX were required to enable detection of actin in 50 fg of RNA without the use of IVT.

In conclusion, MEX can be used in consort with IVT to extend the sensitivity of the detection, whilst minimizing cycle number. This may be important for increasing the amplicon yield from MEX whilst retaining linearity of representation in the amplicon pool. In addition, the production of RNA run-offs from MEX products allows the use of these products as probes on Affymetrix and other microarrays.

### Example 5 MEX used in Target Identification

To demonstrate the utility of MEX, c-fibres were harvested using LCM from an *in vitro* model of pain. This model was based on primary cultures of rat trigeminal ganglion cells treated with pro-nociceptive agents such as Nerve Growth Factor (NGF).

50 neurons were harvested from trigeminal ganglion cultures and RNA extracted and amplified using 24 cycles of MEX. Gene-specific PCR primers were used to identify a number of housekeeper and pain-associated genes from these cells. More specifically, the amplicon pool was assayed with primers for genes with known association with nociception. When the amplicon pool was assayed prior to, and following, MEX amplification, a distinct difference could be observed. Whilst control genes such as actin and cyclophilin (act and cyc) could be detected in the pre-MEX pool, other less abundant and biologically more important genes for the nociceptive response, i.e. TrkB receptor, PN3, sodium channel Beta 3 subunit, calcitonin gene-related protein, vasointestinal peptide, neuropeptide Y, galanin and tyrosine hydroxylase (PN3, β3, CGRP, VIP, NPY, Gal, and TH) were absent. This was in contrast to the amplicon pool following MEX amplification, where the majority of these pro-nociceptive genes were readily detectable. This also validated the *in vitro* model of pain.

Therefore, MEX is of use in detecting genes of interest in small numbers of pathologically relevant cells. These genes may be used as targets for therapeutic intervention.

### Example 6 MEX used in library construction

The MEX primers have been designed to be compatible with many downstream applications. These include use in the manufacture of subtracted PCR libraries.

MEX products from small numbers of cells (100ng total RNA equivalent) were amplified from trigeminal ganglion primary cultures treated with or without nerve growth factor and purified to produce the starting material for the manufacture of subtracted libraries. These were size-selected, to remove small products and primer sequences (<50 base pairs). Linker molecules were ligated to these products to facilitate the subtraction (these were quality controlled by using combinations of linker-based/gene-based PCR primers) and two rounds of suppression PCR subtraction applied to the MEX amplicons. This produced the final forward and reverse-subtracted library pairs. The libraries were quality controlled to ensure that "housekeeper" genes common to both subtraction pools had been removed during the subtraction process. This was demonstrated by the removal of actin transcripts from the subtracted libraries. As a final check of the content of these libraries, products were cloned and sized. A number of these clones were sequenced and differential expression was identified.

Therefore, MEX can be used to analyse gene expression in small numbers of cells and to identify genes that are differentially expressed between two or more cell types or cell types with distinct phenotypes.

### Example 7

MEX was used to amplify a dilution series of cDNA from MEX reverse transcribed total RNA (2 ng-2 µg) using a range of PCR cycles from 15 to 25 cycles. In parallel, conventionally reverse transcribed RNA of equivalent concentration was similarly amplified. PCR amplicons were only seen after MEX amplification. MEX amplification was successful (as determined by the presence of visible products on agarose gels) from as little as 20 ng of total RNA when combined with 25 cycles of PCR.

Further, in comparison of amplicons following MEX amplification of 100 ng of total RNA with 10 µg unamplified total RNA, the size range of products after MEX amplification is similar to those in the unamplified control sample. In addition, the quantity of product in the MEX samples is greater than in the amplified material despite starting from 1% of the control sample.

### Example 8

A dilution of RNA was amplified to stage one MEX and aliquots of these MEX products used as templates in an IVT reaction using T3 RNA polymerase. Using this approach, the sensitivity of MEX can be extended by 3 orders of magnitude, from ~10 ng tRNA to as little as 2.5 pg RNA. Strand-specific probes can be made using this technique. T3 RNA polymerase can be used to make sense-strand RNA, whilst T7 can be used to synthesize anti-sense transcripts.

The orientation of these IVT products was analysed by screening microarrays containing strand-specific probes, i.e. with sense and anti-sense probes separately coupled at different coordinates to the glass slide. Given that aliquots from the same Stage 1 reaction were used as template for these sense and anti-sense reactions, a clear difference in the hybridization patterns of the two target preparations would be expected if faithful sense and anti-sense probes were being produced, as predicted.

More specifically, Stage 1 MEX products were transcribed *in vitro* using either T3 or T7 RNA polymerase and the RNA reverse transcribed and labeled with cy3(T7) or cy5 (T3) prior to hybridization to a strand-specific microarray. The hybridisation signal is entirely "green" (cy 3) only or "red" (cy 5) only, with few if any "yellow" spots, which would indicate inappropriate transcription of mixed, sense/antisense transcripts.

In order to illustrate that strand-specific transcripts were produced from Stage 1 MEX products, T3 and T7 IVT run off targets were produced and each labeled with cy 3 and cy 5. These were hybridized together (T3 cy3 v T3 cy5 and T7 cy3 v T7cy5) to microarrays comprised of strand specific probes. The hybridisation intensities to both sense and anti-sense probes to tubulin were compared across each microassay. Results showed that T3 targets hybridized to sense strand probes, giving a higher fluorescent signal, when compared to anti-sense probes. This was reversed when T7 targets were used. This illustrates that strand specific hybridisation is occurring. The difference in intensity between the sense and antisense probes for tubulin in the T₃ experiment was 10³-fold and 10⁴-fold in the T₇.

### Example 9

To quantify the level of amplification achieved during MEX, the quantity ofRNA produced from a typical MEX reaction was measured. -45 µg of polyA RNA was synthesized from <5 ng of input total RNA (~50 pg polyA RNA). Therefore, approximately 100,000 fold amplification had been achieved. This gives a crude measure of the level of amplification possible, although this is likely to be an under-estimate, due to the exhaustion of key reaction components during the amplification process. In parallel, the relative enrichment of genes within the amplicon pool was ascertained by real-time quantitative RT-PCR.

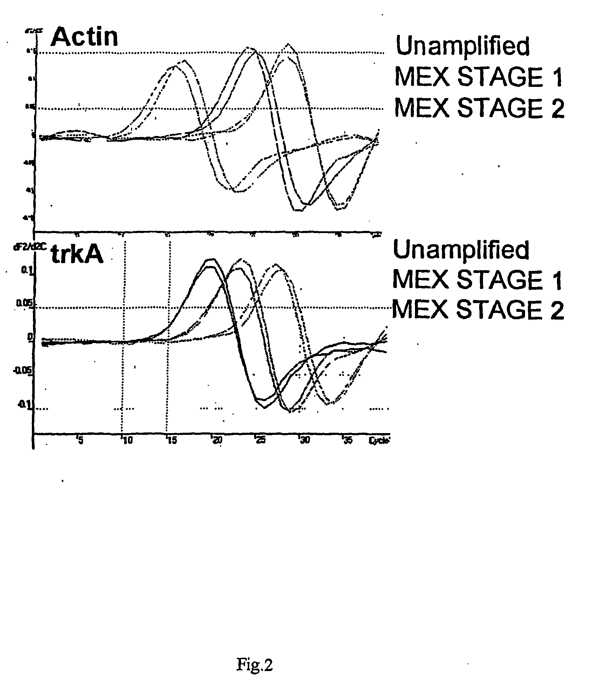

More particularly, real-time quantitative PCR was performed for two genes, actin and the high affinity NGF receptor trkA, on two balanced cDNA samples prior to MEX, after stage 1 MEX and after stage 2 MEX. The two graphs of Figure 2 depict PCR take-offpoints as determined by Sybr green assay. The two samples maintain their relative levels of these two transcripts through the amplification process, although the relative cycle number at which these appear is shifted to the left, indicating amplification of the cDNA pool has occurred, resulting in an earlier detection of the transcripts.

### Example 10

Subtracted MEX products and conventionally produced libraries were cloned and amplified for use as probes on an in-house constructed microarray. This microarray was screened using paired MEX produced subtracted libraries. A substantially linear relationship was seen in a graphical interpretation of the hybridization signal, where each probe is depicted as a single data point with its intensity of cy3 fluorescence shown on the y axis and cy 5 fluorescence on the x axis.

### Example 11

Two independently reverse transcribed RNAs from the same RNA source were amplified by MEX and labeled independently with either cy3 or cy5. These samples were mixed and hybridized to a microarray. The "yellow" nature of all the probes post-hybridisation is an indication that the signal in each channel was equal. This is shown when each probe was plotted according to its signal in the cy3 (y axis) and cy 5 (x axis) channels; the straight-line nature of the graph is a demonstration that identical RNA samples are amplified identically in separate MEX reactions. This is the predicted result as the starting material amplified/labeled in each reaction/channel was identical. This illustrates that MEX maintains sample RNA representation during amplification.

### Example 12

Two independently reverse-transcribed RNAs from two separate sources (brain cy3 and testis cy5) were compared following MEX amplification or pre-amplification. The hybridization pattern was almost identical when these samples were compared, illustrating that MEX had maintained the representative balance between the two RNA sources, whether amplified or unamplified.

### Example 13

Quantities of RNA from independent reverse transcriptions of the same RNA source between 1 µg and 1 ng were amplified by MEX and IVT amplifications and labeled by either cy 3 or cy 5. Quantities are as follows:

| | | |
|---|---|---|
| 1 = 1 µg starting total brain RNA RT | 250 ng MEX | 25 ng MEX/IVT |
| 2 = 10 ng starting total brain RNA RT | 2.5 ng MEX | 250 pg ME3X/IVT |
| 3 = 1 ng starting total brain RNA RT | 250 pg MEX | 25 pg MEX/IVT |

These quantities relate to amount of original sample used for the individual experiment (i.e. ¼ of the RT was used in the MEX reaction and 1/10 ofthe MEX reaction in the IVT). The straight-line relationships between the samples are indicative that representation has been maintained even at extremely low concentrations of input RNA.

## Claims

1. A method for increasing the number of polynucleotides containing sequences corresponding to a mRNA species present in a sample, the method comprising the **steps of:**
(i) in a single step, reverse transcription of the mRNA species using a heeled 5'-amplification primer and a heeled 3'-amplification primer, each heeled primer comprising a heel sequence including a unique sequence, and either or each heel sequence includes a RNA polymerase promoter site, and the heeled 5'-amplification primer includes a variable sequence, and the heeled 3' amplification primer primes at the 3' polyA site in the transcripts whereby the RNA is reverse-transcribed to produce double-stranded cDNA and then multiple cDNAs according to the variable sequence; and
(ii) amplification of the cDNA using primers sufficiently identical to the unique sequences within the heel sequences of the heeled 5'-amplification primer and the heeled 3'-amplification primer.

2. A method according to claim 1, which additionally comprises the step of:
(iii) *in vitro* transcription, to produce RNA run-offs from either end of the amplicons.

3. A method according to claim 1 or claim 2, wherein each heel sequence includes a different RNA polymerase site.

4. A method according to claim 3, for the production of a strand-specific library.

5. A method according to any preceding claim, for the production of a subtracted library from two cell populations.

6. A method according to any preceding claim, which comprises cloning the polynucleotide products and immobilising them in an array.

7. A method according to any preceding claim, wherein the sample is from laser capture microdissection.

8. A method according to any preceding claim, wherein the sample is from patch clamp harvesting.

9. A method according to any preceding claim, wherein the heel sequence of the heeled 5'-amplification primer and/or the heeled 3'-amplification primer includes the nucleotide sequence of a cleavage site.

10. A method according to claim 9, wherein the cleavage site is located at the 3' end of its heel sequence.

11. A method according to claim 10, wherein the heel sequence of the heeled 5'-amplification primer and the heel sequence of the heeled 3'-amplification primer have identical cleavage sites.

12. A method according to claim 10, wherein the heel sequence of the heeled 5'-amplification primer and the heel sequence of the heeled 3'-amplification primer have different cleavage sites.

13. A method according to any of claims 9 to 12, which comprises the additional step of treating the polynucleotides with an agent that cleaves at the cleavage site.

14. A method according to any preceding claim, wherein amplification comprises up to 50 amplification cycles.

15. A method according to claim 14, wherein each amplification cycle comprises the steps of :
(i) obtaining single-stranded DNA molecules at between 85°C and 97°C;
(ii) annealing the single-stranded DNA molecules at between 45°C and 65°C; and
(iii) elongating the annealed DNA molecules at between 70°C and 75°C.

16. A method according to any preceding claim, which additionally comprises confirming the presence of at least one nucleic acid sequence contained in the reaction mixture after amplification.

17. A method according to claim 17, wherein the confirming comprises any one of:
(i) detection of sequences of interest with specific oligonucleotide probes;
(ii) amplification of sequences of interest with specific oligonucleotide primers; and
(iii) cloning of the DNA molecules obtained in a replication and/or expression vector.

## Patentansprüche

1. Verfahren zum Erhöhen der Anzahl von Polynukleotiden, welche Sequenzen, welche einer in einer Probe vorhandenen mRNA-Spezies entsprechen, enthalten, wobei das Verfahren die Schritte umfasst:
(i) in einem einzelnen Schritt, einer Umkehrtranskription der mRNA-Spezies unter Verwendung eines 5'-Amplifizierungsprimers mit als "heel" bezeichnetem Sequenzüberhang und eines 3'-Amplifizierungsprimers mit als "heel" bezeichnetem Sequenzüberhang, wobei jeder Primer mit Sequenzüberhang ("heeled primer") eine Überhangsequenz ("heel sequence"), welche eine einmalig vorkommende Sequenz umfasst, umfasst und wobei eine oder jede der Überhangsequenzen eine RNA-Polymerase-Promotorstelle umfasst und wobei der 5'-Amplifizierungsprimer mit als "heel" bezeichnetem Sequenzüberhang eine variable Sequenz umfasst und der 3'-Amplifizierungsprimer mit als "heel" bezeichnetem Sequenzüberhang an der 3'-polyA-Stelle in den Transkripten seine Primerwirkung ausübt, wobei die RNA revers transkribiert wird, um doppelsträngige cDNA und dann eine Mehrzahl von cDNAs entsprechend der variablen Sequenz herzustellen; und
(ii) einer Amplifizierung der cDNA unter Verwendung von Primern, die ausreichend identisch zu den einmalig vorkommenden Sequenzen innerhalb der Überhangsequenzen des 5'-Amplifizierungsprimers mit als "heel" bezeichnetem Sequenzüberhang und des 3'-Amplifizierungsprimers mit als "heel" bezeichnetem Sequenzüberhang sind.

2. Verfahren nach Anspruch 1, welches zusätzlich den Schritt umfasst:
(iii) einer *in vitro*-Transkription, um RNA-Run-off-Transkripte von einem der beiden Enden der Amplicons herzustellen.

3. Verfahren nach Anspruch 1 oder Anspruch 2, wobei jede Überhangsequenz eine unterschiedliche RNA-Polymerase-Stelle umfasst.

4. Verfahren nach Anspruch 3 für die Herstellung einer strangspezifischen Bibliothek.

5. Verfahren nach einem der vorangegangenen Ansprüche für die Herstellung einer Subtraktions-Gen-Bibliothek von zwei Zellpopulationen.

6. Verfahren nach einem der vorangegangenen Ansprüche, welches umfasst, die Polynukleotidprodukte zu klonieren und diese in einer Anordnung ("Array") zu immobilisieren.

7. Verfahren nach einem der vorangegangenen Ansprüche, wobei die Probe aus einer "Laser Capture Microdissection" stammt.

8. Verfahren nach einem der vorangegangenen Ansprüche, wobei die Probe aus einer mittels der Patch-Clamp-Technik erfolgten Materialgewinnung ("patch clamp harvesting") stammt.

9. Verfahren nach einem der vorangegangenen Ansprüche, wobei die Überhangsequenz des 5'-Amplifizierungsprimers mit als "heel" bezeichnetem Sequenzüberhang und/oder des 3'-Amplifizierungsprimers mit als "heel" bezeichnetem Sequenzüberhang die Nukleotidsequenz einer Spaltstelle umfasst.

10. Verfahren nach Anspruch 9, wobei die Spaltstelle sich an dem 3'-Ende von dessen Überhangsequenz befindet.

11. Verfahren nach Anspruch 10, wobei die Überhangsequenz des 5'-Amplifizierungsprimers mit als "heel" bezeichnetem Sequenzüberhang und die Überhangsequenz des 3'-Amplifizierungsprimers mit als "heel" bezeichnetem Sequenzüberhang identische Spaltstellen aufweisen.

12. Verfahren nach Anspruch 10, wobei die Überhangsequenz des 5'-Amplifizierungsprimers mit als "heel" bezeichnetem Sequenzüberhang und die Überhangsequenz des 3'-Amplifizierungsprimers mit als "heel" bezeichnetem Sequenzüberhang unterschiedliche Spaltstellen aufweisen.

13. Verfahren nach einem der Ansprüche 9 bis 12, welches den zusätzlichen Schritt umfasst, die Polynukleotide mit einem Mittel, welches an der Spaltstelle schneidet, zu behandeln.

14. Verfahren nach einem der vorangegangenen Ansprüche, wobei die Amplifizierung bis zu 50 Amplifizierungszyklen umfasst.

15. Verfahren nach Anspruch 14, wobei jeder Amplifizierungszyklus die Schritte umfasst:
(i) einzelsträngige DNA-Moleküle bei zwischen 85°C und 97°C zu erhalten;
(ii) die einzelsträngigen DNA-Moleküle bei zwischen 45°C und 65°C in Form eines "Annealing" zu hybridisieren; und
(iii) die in Form eines "Annealing" hybridisierten DNA-Moleküle bei zwischen 70°C und 75°C zu verlängern.

16. Verfahren nach einem der vorangegangenen Ansprüche, welches zusätzlich umfasst, das Vorhandensein von wenigstens einer Nukleinsäuresequenz, welche in der Reaktionsmischung nach der Amplifizierung enthalten ist, zu bestätigen.

17. Verfahren nach Anspruch 17, wobei das Bestätigen ein beliebiges umfasst von:
(i) Detektion von Sequenzen von Interesse mit spezifischen Oligonukleotidsonden;
(ii) Amplifizierung von Sequenzen von Interesse mit spezifischen Oligonukleotidprimern; und
(iii) Klonieren der erhaltenen DNA-Moleküle in einen Replikations- und/oder Expressionsvektor.

## Revendications

1. Méthode destinée à augmenter le nombre de polynucléotides contenant des séquences correspondant à une espèce d'ARNm présente dans un échantillon, la méthode comprenant les étapes suivantes :
(i) en une seule étape, transcription inverse de l'espèce d'ARNm en utilisant une amorce d'amplification ancre en 5' et une amorce d'amplification ancre en 3', chaque amorce ancre comprenant une séquence ancre comportant une séquence unique, la ou les deux séquence(s) ancre(s) comporte(nt) un promoteur de l'ARN polymérase, et l'amorce d'amplification ancre en 5' comporte une séquence variable, l'amorce d'amplification ancre en 3' s'amorce au niveau du site polyA en 3' dans les produits de la transcription, moyennant quoi l'ARN subit une transcription inverse pour produire de l'ADNc double brin puis des ADNc multiples en fonction de la séquence variable ; et
(ii) amplification de l'ADNc en utilisant des amorces suffisamment identiques aux séquences uniques dans les séquences ancres de l'amorce d'amplification ancre en 5' et de l'amorce d'amplification ancre en 3'.

2. Méthode selon la revendication 1, qui comprend en outre l'étape suivante :
(iii) transcription *in vitro,* pour produire des fuites d'ARN à partir de l'une des extrémités des amplicons.

3. Méthode selon la revendication 1 ou la revendication 2, dans laquelle chaque séquence ancre comporte un site d'ARN polymérase différent.

4. Méthode selon la revendication 3, pour la production d'une banque spécifique de brin.

5. Méthode selon l'une quelconque des revendications précédentes, pour la production d'une banque soustraite de deux populations cellulaires.

6. Méthode selon l'une quelconque des revendications précédentes, qui comprend le clonage des produits polynucléotidiques et leur immobilisation dans une structure.

7. Méthode selon l'une quelconque des revendications précédentes, dans laquelle l'échantillon provient d'une microdissection par capture laser.

8. Méthode selon l'une quelconque des revendications précédentes, dans laquelle l'échantillon est issu d'une collecte par la technique du patch-clamp.

9. Méthode selon l'une quelconque des revendications précédentes, dans laquelle la séquence ancre de l'amorce d'amplification ancre en 5' et/ou de l'amorce d'amplification ancre en 3' comprend la séquence nucléotidique d'un site de clivage.

10. Méthode selon la revendication 9, dans laquelle le site de clivage est situé à l'extrémité 3' de sa séquence ancre.

11. Méthode selon la revendication 10, dans laquelle la séquence ancre de l'amorce d'amplification ancre en 5' et de la séquence ancre de l'amorce d'amplification ancre en 3' ont des sites de clivage identiques.

12. Méthode selon la revendication 10, dans laquelle la séquence ancre de l'amorce d'amplification ancre en 5' et de la séquence ancre de l'amorce d'amplification ancre en 3' ont des sites de clivage différents.

13. Méthode selon l'une quelconque des revendications 9 à 12, qui comprend l'étape supplémentaire consistant à traiter les polynucléotides avec un agent qui se clive au niveau du site de clivage.

14. Méthode selon l'une quelconque des revendications précédentes, dans laquelle l'amplification comprend jusqu'à 50 cycles d'amplification.

15. Méthode selon la revendication 14, dans laquelle chaque cycle d'amplification comprend les étapes consistant à :
(i) obtenir des molécules d'ADN simple brin entre 85°C et 97°C ;
(ii) anneler les molécules d'ADN simple brin entre 45°C et 65°C ; et
(iii)allonger les molécules d'ADN annelées entre 70°C et 75°C.

16. Méthode selon l'une quelconque des revendications précédentes, qui comprend en outre la confirmation de la présence d'au moins une séquence d'acide nucléique contenue dans le mélange réactionnel après l'amplification.

17. Méthode selon la revendication 17, dans laquelle la confirmation comprend l'une quelconque des étapes suivantes :
(i) la détection des séquences d'intérêt avec des sondes oligonucléotidiques spécifiques ;
(ii) l'amplification des séquences d'intérêt avec des amorces oligonucléotidiques spécifiques ; et
(iii)le clonage des molécules d'ADN obtenues dans un vecteur de réplication et/ou d'expression.


### SEQUENCE LISTING

<110> Cambridge Biotechnology Ltd.
<120> NUCLEIC ACID AMPLIFICATION METHOD
<130> REP07135WO
<140> not yet known
   <141> 2003-07-10
<160> 2
<170> PatentIn Ver. 2.1
<210> 1
   <211> 55
   <212> DNA
   <213> Artificial Sequence
<220>
   <223> Description of Artificial Sequence: Oligonucleotide
<400> 1
   actccgggaa ccatagtggc actcctaatt aaccctcact aaagggagat cgtac 55
<210> 2
   <211> 53
   <212> DNA
   <213> Artificial Sequence
<220>
   <223> Description of Artificial Sequence: Oligonucleotide
<400> 2
   caagcattca gtatctcgac tgtaatacga ctcactatag ggagagatcg tac 53